# EUROPEAN PATENT SPECIFICATION

(11) **EP 2 142 096 B1**
(45) Date of publication and mention of the grant of the patent: **09.01.2013**
(21) Application number: 08745118.3
(22) Date of filing: 04.04.2008
(51) Int. Cl.: A61B 5/145, A61B 5/00

(54) **ANALYTE SENSING DEVICE HAVING A PLURALITY OF SENSING ELECTRODES**
ANALYTERFASSUNGSVORRICHTUNG MIT MEHREREN ERFASSUNGSELEKTRODEN
DISPOSITIF DE DÉTECTION DE SUBSTANCE À ANALYSER AYANT PLUSIEURS ÉLECTRODES DE DÉTECTION

(30) Priority: 04.04.2007 US 910013 P
(43) Date of publication of application: 13.01.2010
(73) Proprietor: Isense Corporation, Wilsonville, OR 97070 (US)
(72) Inventor: SASS, Richard, G., Wilsonville, OR 97070 (US); WARD, W., Kenneth, Wilsonville, OR 97070 (US); BRUCE, Robert, Wilsonville, OR 97070 (US); KAPLAN, Daniel, M., Wilsonville, OR 97070 (US)
(74) Representative: Linhart, Angela
(86) International application number: PCT/US2008/059417
(87) International publication number: WO 2008/124597

(56) References cited:
- WO-A1-02/49507
- WO-A1-98/46124
- WO-A1-2005/051183
- WO-A2-98/58250
- WO-A2-2006/062668
- US-A- 4 953 552
- US-A- 6 080 116

## Description

### Technical Field

Embodiments of the present invention relate to the field of medical devices, and, more specifically, to an electrochemical device having one or more sensing electrodes.

### Background

There are several instances of medically useful devices which are mechanically slender and are inserted through the skin. For example, sensors facilitate the sensing of certain conditions within a patient. Electrochemical sensors are commonly used to monitor blood glucose levels in the management of diabetes. In one scheme, a single electrochemical sensor incorporating an enzyme is fabricated onto a small diameter wire. A second reference electrode is also fabricated around the wire near the sensing electrode. The sensor assembly is inserted through the skin into subcutaneous tissue so that it is surrounded by interstitial fluid. A portion of the sensor assembly exits the skin, remaining outside the body, where electrical connections to the sensing electrode and reference electrode may be made. A suitable electronic measuring device outside the body may be used to measure electrical current from the sensor for recording and displaying a glucose value. These types of devices are described, for example, in US Patent No. 5,965,380 to Heller et al. and US Patent No. 5,165,407 to Wilson et al.

WO 02/49507 A1 discloses a glucose sensor in the form of a skin patch with a microneedle. The fluid drawn onto the patch is selectively switched between a number of microchannels by means of electroosmotic pumps and hydrophobic gates, wherein each microchannel has an electrochemical detector for sensing glucose concentrations.

WO 2006/062668 A2 discloses an electrical biosensor consisting of two or more needle-shaped electrodes for monitoring of molecules such as glucose in the subcutaneous tissue. The two electrodes extend perpendicularly from a sensor base which provides skin fixation and electric connection, wherein one electrode is used as the sensing electrode and the other as the reference electrode.

WO 2005/051183 A1 discloses a needle sensor for subcutaneous measurement with one electrode also serving as a cutting device and at least one further electrode which is displaceable from one position in which the end of the further electrode is flush or behind the end of the cutting device to an advanced position.

### Brief Description of the Drawings

Embodiments of the present invention will be readily understood by the following detailed description in conjunction with the accompanying drawings. Embodiments of the invention are illustrated by way of example and not by way of limitation in the figures of the accompanying drawings.

Figure 1 illustrates an exemplary analyte sensing device;

Figures 2A, 2B, and 2C illustrate an exemplary analyte sensing device in a partially exploded view (Figure 2A), a partial perspective view (Figure 2B), and an assembled perspective view (Figure 2C);

Figures 3A, 03B, 3C, and 3D illustrate lower surface features of an analyte sensing device base;

Figure 4 illustrates a sensing electrode insertion aid;

Figure 5 illustrates an exploded view of an analyte sensing device, a sensing electrode insertion aid, and an associated overbandage;

Figures 6A and 6B illustrate an exemplary analyte sensing device with a plurality of sensing electrodes in accordance with various embodiments of the present invention;

Figures 7A, 7B, 7C, and 7D illustrate various configurations of sensing electrodes in accordance with various embodiments of the present invention;

Figures 8A and 8B illustrate various configurations of sensing electrodes in accordance with various embodiments of the present invention;

Figure 9 illustrates an exemplary analyte sensing system in accordance with various embodiments of the present invention; and

Figures 10A and 10B illustrate an exemplary embodiment of an analyte sensing device in which a main body is movable or slidable with respect to an outer support sleeve.

### Detailed Description of Embodiments of the Invention

In the following detailed description, reference is made to the accompanying drawings which form a part hereof, and in which is shown by way of illustration embodiments in which the invention may be practiced. It is to be understood that other embodiments may be utilized and structural or logical changes may be made without departing from the scope of the present invention. Therefore, the following detailed description is not to be taken in a limiting sense, and the scope of embodiments in accordance with the present invention is defined by the appended claims.

Various operations may be described as multiple discrete operations in turn, in a manner that may be helpful in understanding embodiments of the present invention; however, the order of description should not be construed to imply that these operations are order dependent.

The description may use perspective-based descriptions such as up/down, back/front, and top/bottom. Such descriptions are merely used to facilitate the discussion and are not intended to restrict the application of embodiments of the present invention.

The terms "coupled" and "connected," along with their derivatives, may be used. It should be understood that these terms are not intended as synonyms for each other. Rather, in particular embodiments, "connected" may be used to indicate that two or more elements are in direct physical or electrical contact with each other. "Coupled" may mean that two or more elements are in direct physical or electrical contact. However, "coupled" may also mean that two or more elements are not in direct contact with each other, but yet still cooperate or interact with each other.

For the purposes of the description, a phrase in the form "A/B" or "A and/or B" means "(A), (B), or (A and B)". For the purposes of the description, a phrase in the form "at least one of A, B, and C" means "(A), (B), (C), (A and B), (A and C), (B and C), or (A, B and C)". For the purposes of the description, a phrase in the form "(A)B" means "(B) or (AB)" that is, A is an optional element.

The description may use the phrases "in an embodiment," or "in embodiments," which may each refer to one or more of the same or different embodiments. Furthermore, the terms "comprising," "including," "having," and the like, as used with respect to embodiments of the present invention, are synonymous.

The present invention provides an analyte sensing device according to claim 1 Further preferred embodiments of the invention are defined in the dependent claims.

In embodiments, each sensing electrode may serve as an anode, a cathode, or a combination of an anode and cathode. In embodiments, there may be any suitable number of electrodes, such as 2, 3, 4, 5, 10, 15, 20, 30, 40, or more sensing electrodes.

In an embodiment, a plurality of sensing electrodes may comprise one or moro anodes and one or more cathodes. In an embodiment, a plurality of sensing electrodes may comprise an equal number of anodes and cathodes, or, in an embodiment, there may be an unequal number of anodes and cathodes. In an embodiment, one or more sensing electrodes may comprise both an anode and a cathode on a single sensing electrode. Additional details pertaining to suitable sensing electrodes may be found in US Patent Nos. 5,965,380; 5,165,407; 7,146,202; and US Patent Application No. 10/640,980.

In an embodiment, various sensing electrodes of a plurality of sensing electrodes may be each configured to detect different analytes, or, in an embodiment, each sensing electrode may be configured to detect the same analyte, such as lactate, glucose, etc.

In an embodiment, a plurality of sensing electrodes are provided and may serve as the anode(s), while a connected base unit (main body) may have a skin-contact surface that serves as the cathode.

In an embodiment, there may be provided an analyte sensing device comprising a main body configured to reside on skin of an individual when in use, the main body having one or more electrical components, and an analyte sensing electrode extending substantially perpendicularly from and electrically coupled to the main body, the analyte sensing electrode configured for insertion into the skin of the individual. For the purposes of describing embodiments herein, the phrase "substantially perpendicularly" refers to an angle of orientation that is essentially 90°, although some reasonable degree of departure from 90° is tolerated, such as +/- 5-10°.

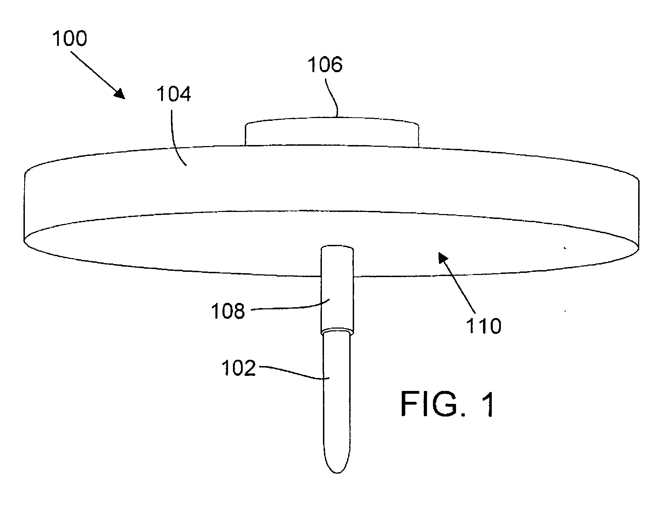

Figure 1 illustrates an exemplary analyte sensing device 100 with a sensing electrode 102. Analyte sensing device 100 also has a main body 104 in one or more parts in which and/or on which may be housed/coupled one more electrical components such as a memory component, a battery component, a transmitter, a receiver, a transceiver, a processor, and/or a display component, etc. One or more electrical components may be provided in/on an associated device (not shown) adapted to be electrically coupled to analyte sensing device 100, such as coupled to main body 104. An insertion device 106 may also be provided, which may comprise one or more parts whether affixed to main body 104, removable from main body 104, or otherwise coupled to main body 104. Insertion device 106 may be a passive guidance mechanism into which or through which sensing electrode 102 may be inserted. Insertion device 106 may have contained therein, or may be couplable to a separate device having, a motive force device, such as a spring, configured to provide a motive force to sensing electrode 102 to drive sensing electrode 102 into skin. Insertion device 106 may be absent, or may be simply a location where a user (or another individual) may press to manually insert sensing electrode 102 into skin.

Sensing electrode 102 may be coated along a portion of its length by a layer of dielectric 108, such as constructed of polyimide. Sensing electrode 102 may be an anode. Sensing electrode 102 may be both an anode and a cathode. For example, a silver or silver/silver chloride wire coil or sleeve may be disposed on/around a portion of dielectric 108 to serve as a reference electrode. Part of or the entire lower surface 110 of main body 104 may be a cathode (reference electrode).

Suitable cathode materials include, but are not limited to, silver or silver/silver chloride. Suitable anode materials include, but are not limited to, platinum, palladium, and gold. A sensing electrode may be a combination anode and cathode such as described in US Patent No. 7,146,202, and US Patent Application No. 10/640,960.

While analyte sensing device 100 is shown with a circular footprint, other shapes may be utilized, whether irregular or regular, such as an oval, square, rectangle, etc. Figure 1 shows sensing electrode 102 exiting main body 104 at a central point of surface 110, although, sensing electrode 102 may exit main body 104 at off-center locations.

Sensing electrode 102 may form a variety of angles with main body 104, including 90°. Other examples may utilize other angles such as 20°, 40°, 60°, or 80°. A benefit of a 90° angle of orientation is that sensing electrode 102 may penetrate to the greatest depth in the skin with a given length electrode. A smaller angle (20°, 40°, etc.) may require using a longer electrode to reach a particular desired depth of penetration, such as to reach subcutaneous tissue. In an example in which sensing electrode 102 is configured at a 90° angle with respect to main body 104, a suitable length of sensing electrode 102 may have approximately 4-6 mm extending outside main body 104. In an example in which sensing electrode 102 is partially covered with dielectric 108, of the 4-6 mm extending out of main body 104, approximately 3 mm of sensing electrode 102 may extend beyond dielectric 108. In an example, when using a different angle of insertion, a longer sensing electrode 102 may be used to provide a desired depth of skin penetration.

Sensing electrode 102 may be electrically coupled to one or more electrical contacts and/or electrical components in, on, or associated with main body 104.

Sensing electrode 102 may be at least partially covered with one or more membranes to manage the amount of oxygen, analyte, and/or other elements/compounds that contact sensing electrode 102. Exemplary suitable membranes may be found in US Patent Nos. 5,165,407 and 6,613,379,

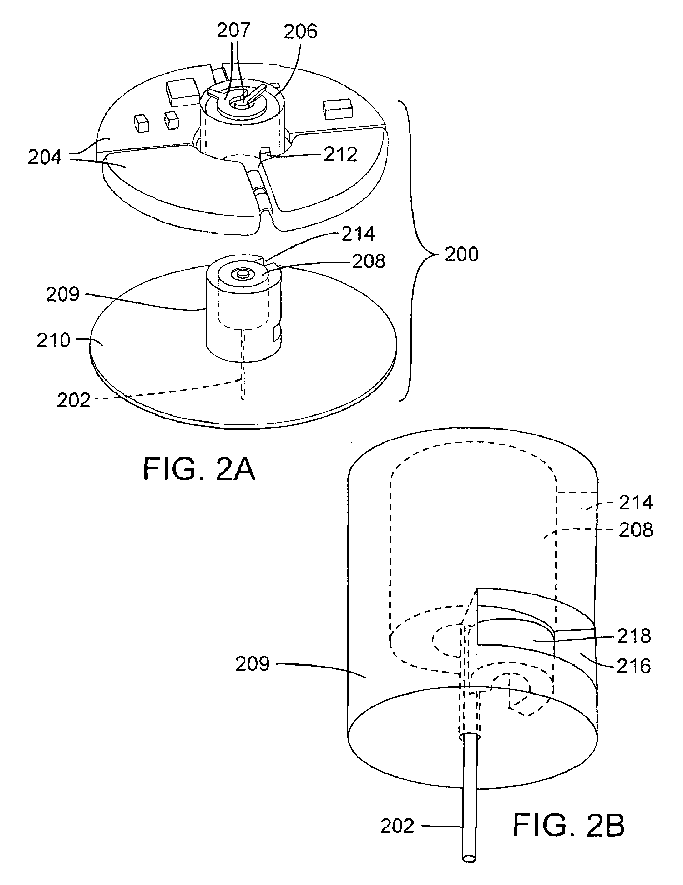

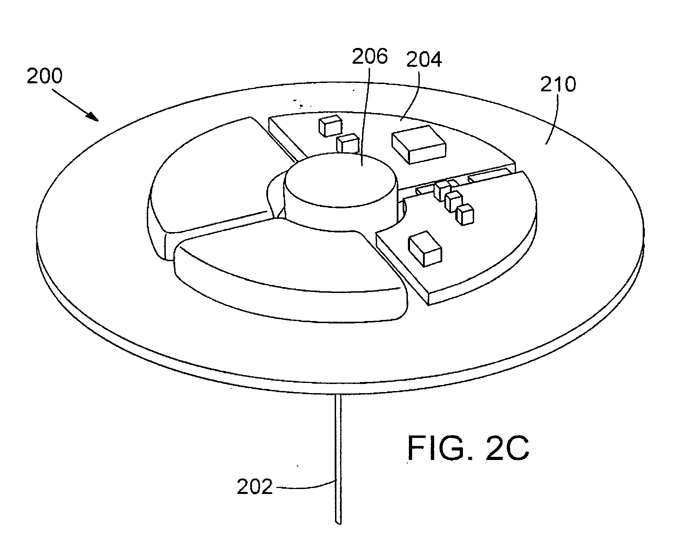

In an example as shown in Figures 2A, 2B, and 2C, an analyte sensing device 200 may have an electronics assembly 204 that may be in one or more portions, such as multiple portions at least partially connected together using a flex cable or other suitable connector(s). Electronics assembly 204 may have one more electrical components such as a memory component, a battery component, a transmitter, a receiver, a transceiver, a processor, and/or a display component, etc. The particular orientation of electronics assembly 204 being substantially in a circular, low-profile arrangement is exemplary. In embodiments, other arrangements may be utilized.

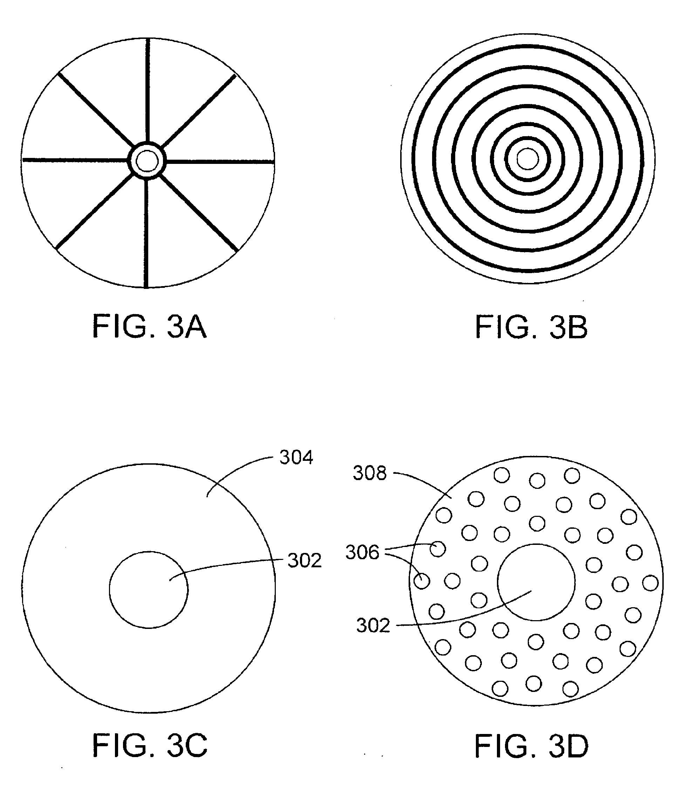

Electronics assembly 204 may be associated with a base 210. In an embodiment, electronics assembly 204 may be coupled to base 210 using adhesive or an integrated mechanism (snap, press-fit arrangement, mechanical guide/track, etc.) that allows for coupling and decoupling of electronics assembly 204 and base 210. Base 210 may provide one or more features such as mechanical support for electronics assembly 204, adhesive coupling to the skin surface of an individual, and/or serving as a reference electrode. Thus, base 210 may be a flexible substrate, such as a bandage. Base 210 may have an adhesive lower surface, which, if present, may be covered at least partially by a removable release liner prior to use. Adhesive may be applied to the lower surface of base 210 uniformly or randomly or in a pattern, for example forming channels (Figure 3A) or circumferential circles (Figure 3B). Utilizing adhesive that covers less than the entire lower surface of base 210 allows the skin to more freely sweat without excessive glandular occlusion and/or offers opportunities for water vapor to escape from under the bandage along the channels or through base 210. In an example in which circumferential circles are used, a larger amount of or a stronger type of adhesive may be used for the innermost and/or outermost circles of adhesive as those regions may be most important for securing sensing electrode 202 in skin (to avoid pistoning or other movement(s) of the electrode) and for maintaining device 200 on the skin. Adhesive applied to the lower surface of base 210 may have embedded/dispersed therein a silver/silver chloride additive to serve as a reference electrode. Wherein including adhesive or not, the lower surface of base 210 may serve as a reference electrode.

Figure 3C illustrates an additional embodiment of a lower surface arrangement of a base (such as base 210 shown in Figures 2A and 2C). In Figure 3C, sensing electrode 302 is represented by a circle; however, the actual diameter of the sensing electrode may be smaller than the diameter of the illustrated circle. The diameter of the illustrated circle reflects the amount of space allocated to sensing electrode 302. Sensing electrode 302 may be an anode, cathode, or combination anode and cathode. Sensing electrode 302 may be an anode and lower surface (skin contact surface) 304 may be a cathode. Surface 304 may be a cathode by being partially or completely constructed from a suitable material, such as silver/silver chloride, or by having a suitable material applied thereto.

Figure 3D illustrates an additional example of a lower surface arrangement of a base (such as base 210 shown in Figures 2A and 2C) of an analyte sensor assembly. In Figure 3D, sensing electrode 302 is represented by a circle; however, the actual diameter of the sensing electrode may be smaller than the diameter of the illustrated circle. The diameter of the illustrated circle reflects the amount of space allocated to sensing electrode 302. In an embodiment, sensing electrode 302 may be an anode, cathode, or combination anode and cathode. Sensing electrode 302 may be an anode and surface protrusions 306 (small bumps or mounds) may each be a cathode or may collectively be a single cathode. Protrusions 306 may be a cathode(s) by being partially or completely constructed from a suitable material, such as silver/silver chloride, or by having a suitable material applied thereto. Surface 308 may be an adhesive or may have an adhesive applied thereto.

Returning to Figures 2A, 2B, and 2C, coupled to electronics assembly 204 is shown a cap 206 that is configured to couple physically and/or electrically with a corresponding subassembly 208 and subassembly housing 209 associated with base 210. Cap 206 is provided with one or more components to engage with subassembly 208 and housing 209. Cap 206 has a "key" protrusion 212 that is configured to fit within vertical slot 214 and to slide along horizontal slot 216 when cap 206 is rotated about subassembly housing 209. In addition, when cap 206 is rotated and protrusion 212 slides along slot 216, protrusion 212 may bend contact 218 such that contact 218 is moved into electrical contact with electrode 202. Contact 218 then provides an electrical pathway from electrode 202 through contact 218, through one or more portions of cap 206, such as through configured electrical contacts 207, and into electronics assembly 204. Each of the electrical pathways are not shown in detail but may be formed with any suitable material using one or more known or later developed fabrication processes.

Some or all of the components may be configured to be disposable or reusable. Electronics assembly 204 and cap 206 may be configured to be reusable. Base 210, subassembly 208, subassembly housing 209, and electrode 202 may be configured to be disposable.

Cap 206 may be configured to engage with subassembly 208 to move electrode 202 into skin. A separate insertion device may be used to move electrode 202 into skin. Such an insertion device may be mated to subassembly 208 and/or subassembly housing 209 to provide a motive force to subassembly 208 to move electrode 202 into skin. Alternatively, a motive force may be provided manually by a user.

Sensing electrodes may be rigid or flexible. Sensing electrodes may be constructed with blunt or sharpened skin-penetrating distal ends.

To assist in the insertion of a sensing electrode, an analyte sensing device may be provided with a short hollow trocar through which a sensing electrode may pass.

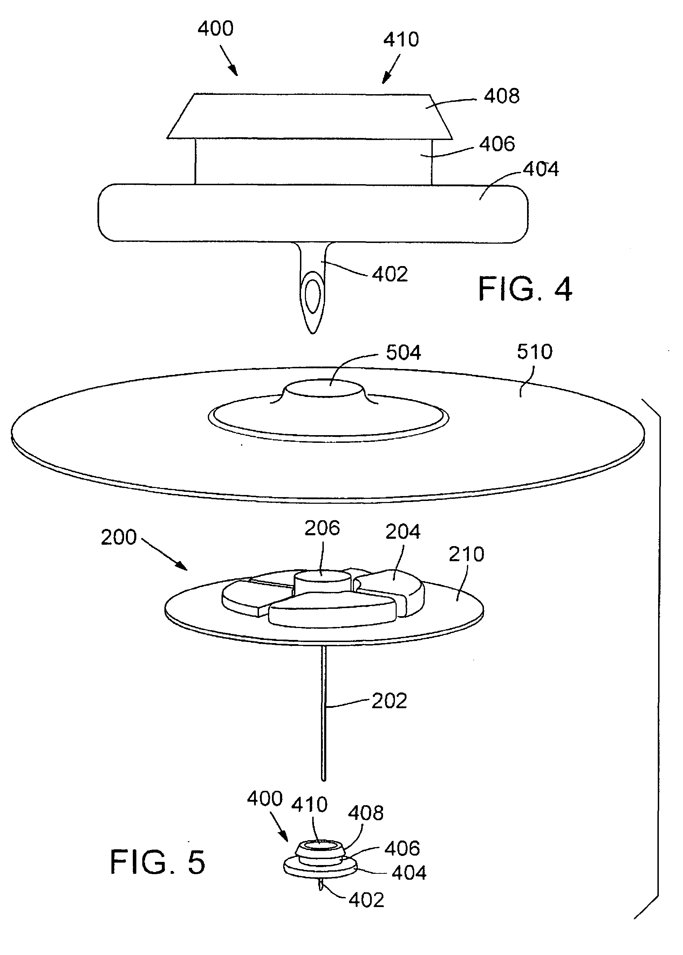

Figure 4 illustrates a sensing electrode insertion aid/device 400 (also referred to herein as a thumbtack) that may provide an initial hole in skin into which a sensing electrode may be simultaneously or subsequently inserted. Device 400 has a short hollow trocar 402 that is relatively sharp to provide for easy penetration of skin to a defined depth. Device 400 may have a base 404 and a docking port 406 to which may be coupled an analyte sensor assembly. Docking port 406 may comprise an extended ridge 408 or other such mechanism that may engage with one or more features on the analyte sensor assembly to aid in securing analyte sensor assembly to device 400. Docking port 406 may also have an opening 410 at the upper surface thereof to accept a sensing electrode and to allow the sensing electrode to pass through docking port 406 and through trocar 402.

Trocar 402 may be constructed of a metal, plastic, etc. Trocar 402 may be constructed of a shape memory material, a thermoplastic, or other material having a temperature-dependent structural integrity such that when inserted into a body, the temperature of trocar 402 may be increased and trocar 402 may soften. A softened trocar 402 may be more comfortable to a patient.

Trocar 402 may be approximately 1-5 mm, such as 2-3 mm, in length.

Thus, there is provided an analyte sensing electrode insertion aid comprising a base configured to reside on skin of an individual when in use, the main body having at least one hole passing from an upper portion to a lower portion of the base, and a hollow trocar extending substantially perpendicularly from and coupled to the base, the trocar providing a hole that couples to the hole of the base such that a passage is provided from the upper portion of the base through the base and through the trocar, the passage configured to directionally guide an analyte sensing electrode into skin of the individual.

Figure 5 illustrates an exploded view of an analyte sensor assembly 200 poised for engagement with a thumbtack device 400. Opening 410 of docking port 406 may be provided with sloped interior walls to assist in guiding sensing electrode 202 into a hole (not shown) to allow sensing electrode 202 to pass through docking port 406 and through trocar 402. Also shown in Figure 5 is an overbandage 510 that may be used to cover analyte sensor assembly 200 to protect various underlying components from moisture, debris, contact, etc. In an embodiment, overbandage 510 may be flexible to account for various underlying contours. In an embodiment, overbandage 510, whether flexible or not, may be configured with one or more integrated/formed extended surface formations 504 that may form a corresponding cavity on a reverse surface of overbandage 510 to accommodate one or more features of the underlying analyte sensor assembly 200.

An overbandage, such as overbandage 510, may have an approximate size of 2-4 inches, such as approximately 3 inches, in diameter. A base (whether in the form of a bandage or not), such as base 210, may have an approximate size of 1-2 inches, such as approximately 1.5 inches, in diameter.

In an embodiment, a plurality of sensing electrode (analyte sensor) is utilized. Such an embodiment may increase the amount of analyte that may be detected at a given time which may be useful if a single sensing electrode has a low sensitivity or a low sensitivity in the particular environment.

In an embodiment, there is provided an analyte sensing device comprising a main body configured to reside on skin of an individual when in use, the main body having one or more electrical components, and a plurality of analyte sensing electrodes extending from and electrically coupled to the main body, the analyte sensing electrodes configured for insertion into the skin of the individual.

In embodiments, when using multiple sensing electrodes, various calculations may be performed on the measured data. In such embodiments, the values from the sensing electrodes may be averaged, various ones may be used while other values may be discarded, calibration may be independent for each sensing electrode, etc.

Thus, there is provided a method for measuring analyte in an individual comprising inserting a portion of an analyte sensing device into skin of an individual, the analyte sensing device comprising a main body configured to reside on the skin of the individual when in use, the main body having one or more electrical components, and a plurality of analyte sensing electrodes extending from and electrically coupled to the main body, the analyte sensing electrodes configured for insertion into the skin of the individual, and measuring an amount of analyte detected directly or indirectly by one or more of the plurality of analyte sensing electrodes. As detection methods may rely on measuring a reaction product as opposed to measuring analyte directly, the phrase "directly or indirectly" is intended to cover measurement of analyte or a proxy for a particular analyte, for example a product of a reaction involving a particular analyte.

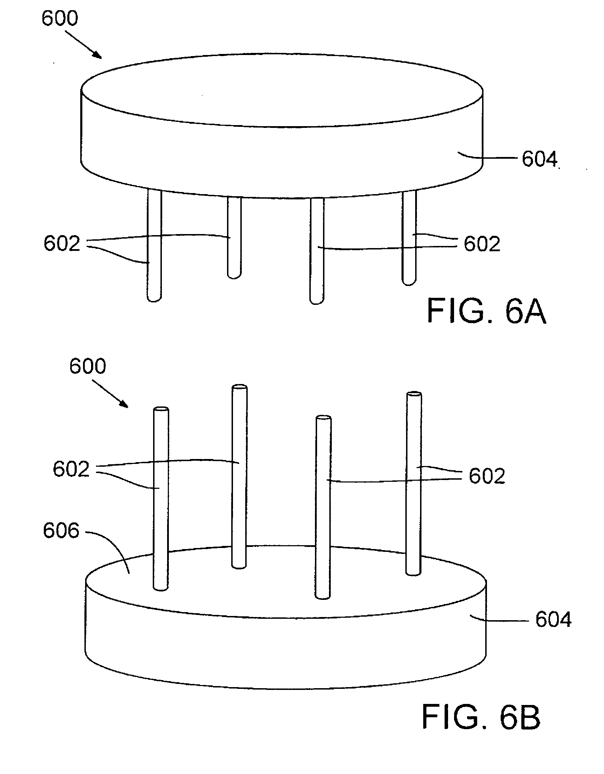

Figures 6A and 6B illustrate an exemplary analyte sensing device 600 with a plurality of sensing electrodes 602. Analyte sensing device 600 also has a main body 604 in which may be housed one more electrical components such as a memory component, a battery component, a transmitter, a receiver, a transceiver, a processor, and/or a display component, etc. In an embodiment, one or more electrical components may be provided in an associated device (not shown) adapted to be electrically coupled to the analyte sensing device.

Each sensing electrode 602 may serve as an anode, a cathode, or a combination of an anode and cathode. Suitable cathode materials include, but are not limited to, silver or silver/silver chloride. In an embodiment, suitable anode materials include, but are not limited to, platinum, palladium, and gold. A sensing electrode 602 may be a combination anode and cathode such as described in US Patent No. 7,146,202, and US Patent Application No. 10/640,980.

Each sensing electrode may be an anode while a connected base unit (main body) 604 may have a skin-contact surface 606 that serves as the cathode.

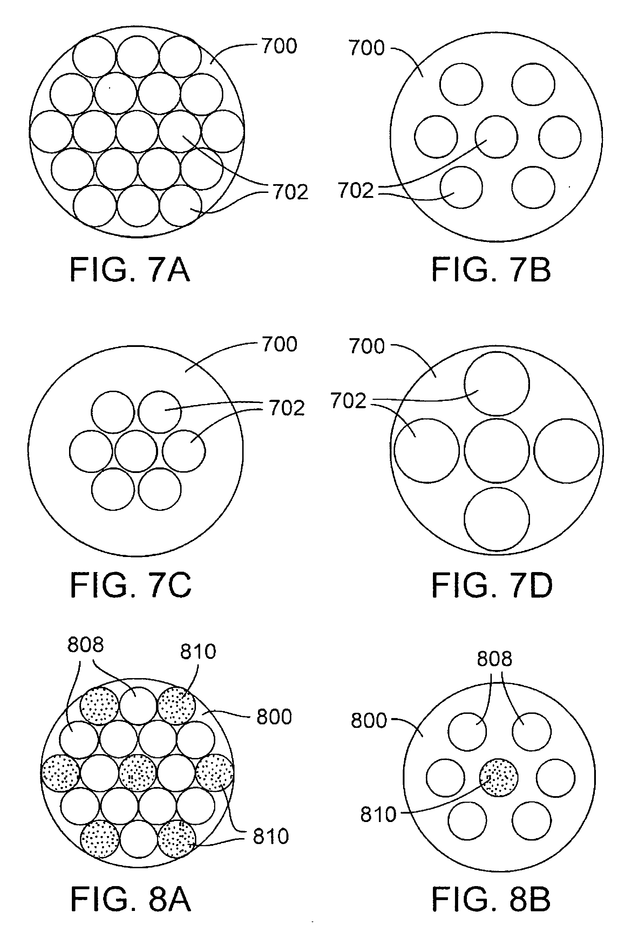

In embodiments such as shown in Figures 7A, 7B, 7C, and 7D, various configurations of sensing electrodes 702 may be utilized. Each sensing electrode 702 is represented by a circle; however, the actual diameter of the sensing electrode is typically smaller than the diameter of the illustrated circle. The diameter of the illustrated circle reflects the amount of space allocated to each sensing electrode 702 to ensure that the sensing electrodes 702 are not too closely packed and to achieve a suitable degree of tissue compression. If the sensing electrodes 702 are too closely packed, an unreasonably high degree of tissue compression may be needed to puncture the skin. The space allocated for each sensing electrode may be approximately 2-3 mm in diameter, whereas the actual diameter of a suitable exemplary sensor may be approximately 100-200µm.

Figure 7A illustrates an example in which an analyte sensing device 700 has nineteen sensing electrodes 702. With the defined allocated space for each sensing electrode 702, Figure 7A shows a maximized number of sensing electrodes 702 for the given space.

Figures 7B and 7C illustrate a less than maximal number of sensing electrodes 702 for the given space (given a predefined allocated space for each electrode). Figure 7B illustrates a loosely arranged set of sensing electrodes 702, whereas Figure 7C illustrates a more densely arranged set of sensing electrodes 702. A more densely arranged set of sensing electrodes 702 generally results in more uniform pressure being applied to each sensing electrode 702.

Figure 7D illustrates a set of sensing electrodes 702 with an allocated space for each sensing electrode 702 that is larger than that shown in Figures 7A, 7B, and 7C. A smaller number of sensing electrodes 702 and/or an increased amount of allocated space for each sensing electrode 702 may result in reduced tissue compression required to insert the sensing electrodes 702 into skin.

In the examples of Figures 7A, 7B, 7C, and 7D, each sensing electrode may independently be an anode, cathode, or a combination anode and cathode. One or more of the sensing electrodes may be an anode and the skin-contact surface of device 700 may be a cathode.

Figures 8A and 8B illustrate anodes 808, such as constructed of platinum, and cathodes 810, such as constructed of silver or silver/silver chloride, coupled to analyte sensing device 800. As may be seen in Figures 8A and 8B, various numbers and configurations of anodes 808 and cathodes 810 may be utilized, whether densely loosely arranged.

The plurality ofsensing electrodes comprises a membrane system or series of layered membranes. Such membranes include an interferent reducing inner layer, an enzyme layer (for example containing glucose oxidase, lactate oxidase, or lactate dehydrogenase), and a permselective outer layer. A silane layer may be provided under and/or over the enzyme layer. Additional details pertaining to suitable membranes may be found in US Patent Nos. 5,165,407 and 6,613,379.

A membrane system or at least one layer of a series of layered membranes may cover all or only a part of the sensing electrodes. A partially covered/surrounded sensing electrode may be constructed, for example, by dip coating, a deposition process, etc. In an embodiment, a bare wire sensing electrode (one without an insulating layer of dielectric) may have a membrane or membrane system covering and surrounding only a portion of the sensing electrode, in particular, covering and surrounding only a portion of the sensing electrode that is intended to reside within the tissue of a user when in use. When using an enzyme layer containing glucose oxidase, one benefit of such an arrangement is to maintain the enzyme layer within the tissue even when the sensing electrode experiences some movement relative to the skin to minimize motion-related artifacts.

A main body may have one or more bare wire anodes with an enzyme layer on a distal portion thereof, and one or more separate wire cathodes. A bare wire anode or cathode may also have at least a portion thereof covered with a dielectric, such as polyimide.

A variety of dimensions of an analyte sensing device may be employed. In an exemplary embodiment, an analyte sensing device having a plurality of sensing electrodes may have a footprint approximately the size of a standard thumbtack or approximately the size of a quarter dollar (approximately 25 mm diameter) or smaller, such as approximately 10 mm.

A plurality of sensing electrodes may each have the same length, or the lengths may vary in order to penetrate the skin to different depths. One or more sensing electrodes may have a length that only penetrates through the epidermis and into the dermis slightly thus picking up the majority of glucose from interstitial fluid in the outer skin layers. A sensing electrode may extend from an associated sensing device by approximately 3 mm to approximately 6 mm. A sensing electrode may extend from an associated sensing device perpendicular to the major surface of the sensing device, or may extend from the sensing device at an angle.

A plurality of sensing electrodes may each have the same outer diameter, or the outer diameters of the sensing electrodes may vary.

A sensing electrode may have an outer diameter of approximately 100-400 microns, or, in an embodiment, approximately 330 microns or smaller.

The distal end (skin contact end) of a sensing electrode may be formed, cut, and/or sharpened to form a variety of shapes of points/ends as desired for ease of insertion. A sensing electrode may have either a blunt distal end or an end that is cut on the bias for easier insertion.

An analyte sensing device may be attached to skin, at least in part, by inserting the associated sensing electrodes into the skin. The analyte sensing device may be inserted manually by a user or another individual. The analyte sensing device may be inserted using an associated insertion device (whether permanent or removably affixed). An associated insertion device may provide a separate motive force, such as with a spring, compressed gas, etc. Additional details regarding suitable insertion devices may be found in US Patent No. 6,695,860 and US Patent Application No. 11/558,394.

It may be difficult to insert the sensing electrodes into skin. Thus, pinching up a fold of skin or pulling the skin tight against the sensing electrodes prior to insertion may ease the insertion efforts.

Additional mechanisms may be provided to secure an analyte sensing device to skin. For example, a sensing device may be inserted into skin of an appendage or another portion of the body, and a flexible or elastic compression band may be further wrapped around the appendage (such as around the upper arm or thigh) or around another portion of the body to hold the analyte sensing device securely against the user's skin. An analyte sensing device may be coupled to an adhesive bandage prior to insertion into the skin, or an adhesive bandage may be first applied to skin and an analyte sensing device as described herein may then be coupled to the bandage and inserted into the skin. An analyte sensing device and/or an associated bandage/patch may also comprise various antimicrobial agents. Additional details regarding adhesive bandages may be found in US Patent Application No. 11/609,768.

An analyte sensing device may be integrated into or onto a bandage such that the user may remove a release liner on the bottom side of the bandage/patch and may press the sensor electrodes into the skin (or with use of an insertion device). The adhesive assists in securing the analyte sensing device on the skin.

In an In an example incorporating an adhesive bandage/patch, the footprint of the coupled components may be, for example, the size of a silver dollar, or approximately 40 mm or smaller, with the analyte sensing device accounting for approximately 40-70%, such as 50-60%, of that footprint.

Each functioning sensing electrode inserted into skin vides a signal indicative of the level of analyte in the measured fluid. In an example having multiple sensing electrodes, the various signals from the sensing electrodes may be averaged to obtain an average analyte value for the analyte sensing device.

Signal averaging software may be used in conjunction with an analyte sensing device having multiple sensing electrodes to account for potential variations in the signals measured from one or more of the sensing electrodes. One or more sensing electrodes may not be functioning properly due to insufficient electrical connections, malfunctioning membranes, insufficient interstitial fluid found in the region in which the particular electrode resides, etc. Such malfunctioning and/or variation may be addressed by utilizing software-driven mathematical averaging to remove the non-functioning or statistically outlying value(s).

In an example having multiple sensing electrodes, for a variety of reasons such as mentioned above, one or more of the sensing electrodes may be excluded from contributing to the measured analyte value if that sensing electrode is determined to be providing a value that differs sufficiently from an average value of the remaining sensing electrodes. A particular value may be excluded or values measured by a particular sensing electrode may be excluded for a set period of time, or for the period of time the sensing electrode is returning an outlying value. A sensing electrode that has been excluded from the measurement calculations may be rejoined with the other sensing electrodes when it is determined to be producing suitable values.

An analyte sensing device having a plurality of electrodes, or one or more associated devices, may further comprise electronic circuitry and components in any desired structural relationship adapted to, in part, receive an electrical signal from an associated sensor and, optionally, to transmit a further signal, for example to an external electronic monitoring unit that is responsive to the sensor signal. The circuitry and other components may or may not include a printed circuit board, a tethered or wired system, etc. Signal transmission may occur over the air with electromagnetic waves, such as RF communication, or data may be read using inductive coupling. Transmission may be over a wire or via another direct connection.

Additional components may be housed in one or more separate modules that may be coupled to (for example, snapped to, wired to, or in wireless communication with) the analyte sensing device. For example, the separate module may contain a memory component, a battery component, a transmitter, a receiver, a transceiver, a processor, and/or a display component, etc.

An analyte sensing device may have an integrated power source such as a battery, for example a coin-cell battery. The entire top surface of the analyte sensing device may be formed by a battery.

An analyte sensing device and/or any other associated devices/components may be disposable, reusable, or the device(s) may be resposable.

An analyte sensing device may be a disposable unit. Coupled to the analyte sensing device, may be a reusable unit. Such a reusable unit may comprise additional electronics and/or other components that either do not fit into the disposable unit, or are preferably made reusable due to cost. An analyte sensing device having a circular profile may be further encircled by a reusable unit that surrounds the analyte sensing device. An analyte sensing device as described above may be encircled such as by a donut-shaped reusable unit, or may be encircled and capped so that the top of the coupled device is the reusable unit.

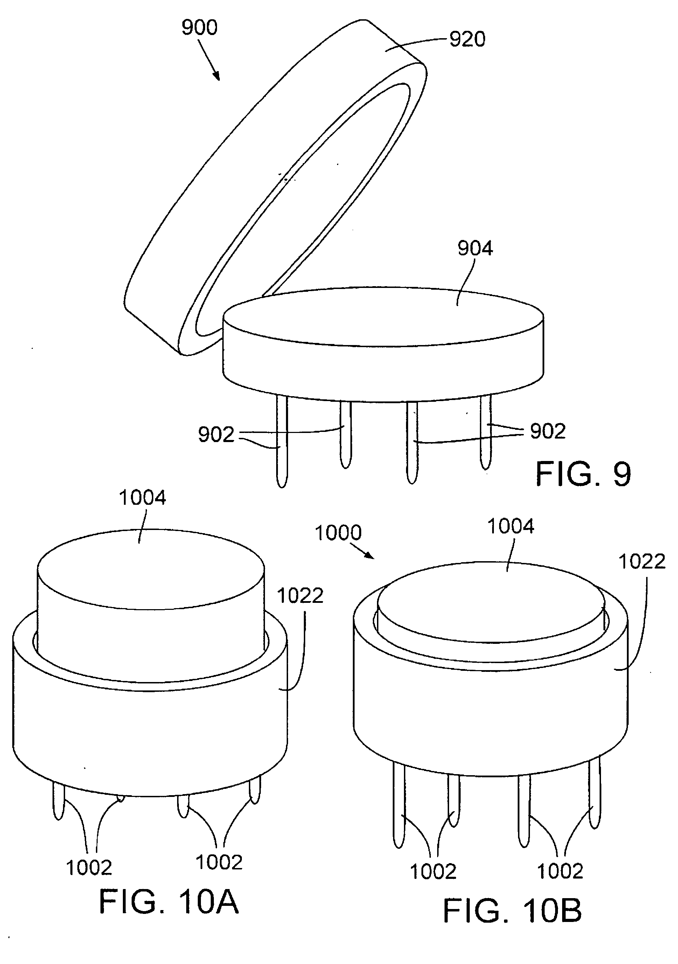

Figure 9 illustrates an exemplary sensing system 900. In an embodiment, system 900 may be resposable. Thus, analyte sensing device body 904 may be disposable and electronics unit 920 may be reusable. Device body 904 may be reusable and electronics unit 920 may be disposable. The reusable portion may contain one or more of the expensive electronic components and/or those that have a usable life longer than that of the sensing electrodes. As indicated in Figure 9, electronics unit 920 may fit over and around analyte sensing device body 904 to electrically couple the components.

Analyte sensing device body 904 further has sensing electrodes 904 extending therefrom.

Sensing electrodes may be coupled to a main body which is movable or slidable with respect to another element which in-turn encloses the sensing electrodes when in a retracted position. The sensing electrodes may then be pushed through one or more openings in the enclosing element to be inserted into skin. The one or more openings may be covered by a film or foil and/or there may be present a bladder or balloon that is pierced during the extension/insertion process.

Figures 10A and 10B illustrate an example of an analyte sensing device 1000 in which a main body 1004 is movable or slidable with respect to an outer support sleeve 1022. Figure 6A illustrates main body 1004 and sensing electrodes 1002 in a partially retracted position, while Figure 10B illustrates main body 1004 and sensing electrodes 1002 in a more extended position simulating the action of the components of sensing device 1000 when sensing electrodes 1002 are inserted into skin.

Outer support sleeve 1022 may contain various electronic components, one or more of which may be coupled to main body 1004, for example using a variety of electrical contacts or flexible wires, etc. Outer support sleeve 1022 may be disposable or reusable. Additional structures may be provided, such as a separate electronics unit (such as element 920 shown in Figure 9).

Within outer support sleeve 1022, channels or guides may be provided to direct sensing electrodes 1002 in the desired direction. In an embodiment, sensing electrodes 1002 may be moved from a completely enclosed or retracted position to a fully extended position by moving main body 1004 with respect to sleeve 1022. A foil, film, or bladder may be provided at the lower base of sleeve 1022 to enclose the bottom of sleeve 1022 and secure sensing electrodes 1002 within sleeve 1022 until such time as sensing electrodes 1002 are pushed through the foil, film, or bladder. Sleeve 1022 may include a series of holes on a lower surface thereof through which electrodes 1002 may pass before being inserted into skin or in which electrodes 1002 may be held/guided.

An insertion force may be provided by a user or by a separate device, such as a spring. Likewise, a retraction force may be provided by a user or a separate device, such as a spring. Suitable ergonomic structures may be incorporated into main body 1004 and/or sleeve 1022 to ease the insertion process and/or to make the use of device 1000 more comfortable for the user.

Thus, there is provided an analyte sensing device comprising a main body having one or more electrical components, the main body having a plurality of analyte sensing electrodes extending therefrom and electrically coupled thereto, and an outer support sleeve having an interior cavity, wherein the main body is movable within the interior cavity and movable with respect to the outer support sleeve from a position in which the plurality of analyte sensing electrodes are fully retracted into the interior cavity to a position in which the plurality of analyte sensing electrodes are extended from the outer support sleeve.

Sensing electrodes in accordance with embodiments herein may be formed by any suitable process including wire machining and deposition/coating processes, chemical etching or other micromechanical processes. The sensing electrodes may be grown as "whiskers" from a silicon chip base.

Although certain embodiments have been illustrated and described herein for purposes of description of the preferred embodiment, it will be appreciated by those of ordinary skill in the art that a wide variety of alternate and/or equivalent embodiments or implementations calculated to achieve the same purposes may be substituted for the embodiments shown and described without departing from the scope of the present invention. Those with skill in the art will readily appreciate that embodiments in accordance with the present invention may be implemented in a very wide variety of ways. This application is intended to cover any adaptations or variations of the embodiments discussed herein. Therefore, it is manifestly intended that embodiments in accordance with the present invention be limited only by the claims.

## Claims

1. An analyte sensing device (600, 1000), comprising:
a main body (604, 1004) configured to reside on skin of an individual when in use, the main body (604, 1004) having one or more electrical components;
**characterized in** the analyte sensing device (600, 1000) further comprising
a plurality of analyte sensing electrodes (602, 1002) extending from and electrically coupled to the main body (604, 1004), the analyte sensing electrodes (602, 1002) configured for insertion into the skin of the individual, each analyte sensing electrode (602, 1002) comprising a membrane system including an interferent reducing inner layer, an enzyme layer, and a permselective outer layer.

2. The analyte sensing device (600, 1000) of claim 1, wherein the plurality of analyte sensing electrodes (602, 1002) are anodes.

3. The analyte sensing device (600, 1000) of claim 1 or 2, wherein a skin-contact surface (606) of the main body (604, 1004) is a cathode.

4. The analyte sensing device (600, 1000) of claim 3, wherein the skin-contact surface (606) of the main body (604, 1004) comprises surface protrusions that comprise the cathode.

5. The analyte sensing device (600, 1000) of one of the claims 1 to 4, wherein the main body (604, 1004) is a flexible substrate.

6. The analyte sensing device (600, 1000) of one of the claims 1 to 5, further comprising a patterned adhesive on a skin-contact surface (606) of the main body (604, 1004).

7. The analyte sensing device (600, 1000) of one of the claims 1 to 5, further comprising adhesive on a skin-contact surface (606) of the main body (604, 1004), wherein the adhesive contains silver/silver chloride.

8. The analyte sensing device (600, 1000) of claim 1, wherein the plurality of analyte sensing electrodes (602, 1002) extend substantially perpendicularly from the main body (604, 1004).

9. The analyte sensing device (600, 1000) of claim 1, wherein the plurality of analyte sensing electrodes (602, 1002) are configured to detect multiple analytes, each analyte sensing electrode (602, 1002) configured to detect a single analyte.

10. An analyte sensing device (600, 1000) of one of the claims 1 to 9, further comprising
an outer support sleeve (1022) having an interior cavity, wherein the main body (604, 1004) is movable within the interior cavity and movable with respect to the outer support sleeve (1022) from a position in which the plurality of analyte sensing electrodes (602, 1002) are fully retracted into the interior cavity to a position in which the plurality of analyte sensing electrodes (602, 1002) are extended from the outer support sleeve (1022).

11. The analyte sensing device (600, 1000) of claim 10, wherein the outer sleeve (1022) further comprises a barrier that must be pierced by the plurality of analyte sensing electrodes (602, 1002) when moved from a fully retracted position to an extended position.

12. The analyte sensing device (600, 1000) of one of the claims 1 to 11, further comprising a signal processor to average signal variation among the plurality of analyte sensing electrodes (602, 1002).

## Patentansprüche

1. Analyterfassungsvorrichtung (600, 1000), umfassend:
einen Hauptkörper (604, 1004), der konfiguriert ist, um sich im Betrieb auf der Haut einer Person zu befinden, wobei der Hauptkörper (604, 1004) ein oder mehrere elektrische Bauteile aufweist;
**dadurch gekennzeichnet, dass** die Analyterfassungsvorrichtung (600, 1000) ferner Folgendes umfasst:
eine Vielzahl von Analyterfassungselektroden (602, 1002), die sich von dem Hauptkörper (604, 1004) aus erstrecken und damit elektrisch gekoppelt sind, wobei die Analyterfassungselektroden (602, 1002) zum Einführen in die Haut der Person konfiguriert sind, wobei jede Analyterfassungselektrode (602, 1002) ein Membransystem umfasst, das eine Störsubstanzen reduzierende Innenschicht, eine Enzymschicht und eine permselektive Außenschicht umfasst.

2. Analyterfassungsvorrichtung (600, 1000) nach Anspruch 1, wobei die Vielzahl von Analyterfassungselektroden (602, 1002) Anoden sind.

3. Analyterfassungsvorrichtung (600, 1000) nach Anspruch 1 oder 2, wobei eine Hautkontaktfläche (606) des Hauptkörpers (604, 1004) eine Kathode ist.

4. Analyterfassungsvorrichtung (600, 1000) nach Anspruch 3, wobei die Hautkontaktfläche (606) des Hauptkörpers (604, 1004) Oberflächenvorsprünge umfasst, welche die Kathode umfassen.

5. Analyterfassungsvorrichtung (600, 1000) nach einem der Ansprüche 1 bis 4, wobei der Hauptkörper (604, 1004) ein flexibles Substrat ist.

6. Analyterfassungsvorrichtung (600, 1000) nach einem der Ansprüche 1 bis 5, ferner umfassend einen gemusterten Klebstoff auf einer Hautkontaktfläche (606) des Hauptkörpers (604, 1004).

7. Analyterfassungsvorrichtung (600, 1000) nach einem der Ansprüche 1 bis 5, ferner umfassend Klebstoff auf einer Hautkontaktfläche (606) des Hauptkörpers (604, 1004), wobei der Klebstoff Silber/Silberchlorid enthält.

8. Analyterfassungsvorrichtung (600, 1000) nach Anspruch 1, wobei sich die Vielzahl von Analyterfassungselektroden (602, 1002) im Wesentlichen rechtwinklig vom Hauptkörper (604, 1004) erstreckt.

9. Analyterfassungsvorrichtung (600, 1000) nach Anspruch 1, wobei die Vielzahl von Analyterfassungselektroden (602, 1002) konfiguriert ist, um mehrere Analyte zu erfassen, wobei jede Analyterfassungselektrode (602, 1002) konfiguriert ist, um einen einzelnen Analyt zu erfassen.

10. Analyterfassungsvorrichtung (600, 1000) nach einem der Ansprüche 1 bis 9, ferner umfassend:
eine äußere Stützhülse (1022), die einen inneren Hohlraum aufweist, wobei der Hauptkörper (604, 1004) innerhalb des inneren Hohlraums bewegbar ist und bezüglich der äußeren Stützhülse (1022) von einer Position, in der die Vielzahl von Analyterfassungselektroden (602, 1002) vollständig in den inneren Hohlraum eingezogen ist, in eine Position, in der die Vielzahl von Analyterfassungselektroden (602, 1002) aus der äußeren Stützhülse (1022) ausgefahren ist, bewegbar ist.

11. Analyterfassungsvorrichtung (600, 1000) nach Anspruch 10, wobei die äußere Hülse (1022) ferner eine Barriere umfasst, die von der Vielzahl von Analyterfassungselektroden (602, 1002) zu durchstechen ist, wenn sie von einer ganz eingezogenen Position in eine ausgefahrene Position bewegt wird.

12. Analyterfassungsvorrichtung (600, 1000) nach einem der Ansprüche 1 bis 11, ferner umfassend einen Signalprozessor, um die Signalvariation in der Vielzahl von Analyterfassungselektroden (602, 1002) zu mitteln.

## Revendications

1. Dispositif de détection d'analytes (600, 1000), comprenant :
un corps principal (604, 1004) configuré pour résider à l'usage sur la peau d'un individu, le corps principal (604, 1004) comportant un ou plusieurs composants électriques ;
**caractérisé en ce que** le dispositif de détection d'analytes (600, 1000) comprend en outre
une pluralité d'électrodes de détection d'analytes (602, 1002) s'étendant depuis et électriquement couplées avec le corps principal (604, 1004), les électrodes de détection d'analytes (602, 1002) étant configurées pour insertion dans la peau de l'individu, chaque électrode de détection d'analytes (602, 1002) comprenant un système de membrane comportant une couche interne réduisant les interférences, une couche d'enzymes, et une couche externe à perméabilité sélective.

2. Dispositif de détection d'analytes (600, 1000) selon la revendication 1, dans lequel la pluralité d'électrodes de détection d'analytes (602, 1002) est constituée d'anodes.

3. Dispositif de détection d'analytes (600, 1000) selon la revendication 1 ou 2, dans lequel une surface de contact avec la peau (606) du corps principal (604, 1004) est une cathode.

4. Dispositif de détection d'analytes (600, 1000) selon la revendication 3, dans lequel la surface de contact avec la peau (606) du corps principal (604, 1004) comprend des saillies superficielles qui comprennent la cathode.

5. Dispositif de détection d'analytes (600, 1000) selon l'une quelconque des revendications 1 à 4, dans lequel le corps principal (604, 1004) est un substrat flexible.

6. Dispositif de détection d'analytes (600, 1000) selon l'une quelconque des revendications 1 à 5, comprenant en outre un adhésif à motif sur une surface de contact avec la peau (606) du corps principal (604, 1004).

7. Dispositif de détection d'analytes (600, 1000) selon l'une quelconque des revendications 1 à 5, comprenant en outre un adhésif sur une surface de contact avec la peau (606) du corps principal (604, 1004), l'adhésif contenant de l'argent/chlorure d'argent.

8. Dispositif de détection d'analytes (600, 1000) selon la revendication 1, dans lequel la pluralité d'électrodes de détection d'analytes (602, 1002) s'étend de façon sensiblement perpendiculaire depuis le corps principal (604, 1004).

9. Dispositif de détection d'analytes (600, 1000) selon la revendication 1, dans lequel la pluralité d'électrodes de détection d'analytes (602, 1002) est configurée pour détecter de multiples analytes, chaque électrode de détection d'analytes (602, 1002) étant configurée pour détecter un seul analyte.

10. Dispositif de détection d'analytes (600, 1000) selon l'une quelconque des revendications 1 à 9, comprenant en outre
un manchon de support externe (1022) comportant une cavité intérieure, le corps principal (604, 1004) étant mobile dans la cavité intérieure et mobile par rapport au manchon de support externe (1022) depuis une position dans laquelle la pluralité d'électrodes de détection d'analytes (602, 1002) sont totalement rétractées dans la cavité intérieure jusqu'à une position dans laquelle la pluralité d'électrodes de détection d'analytes (602, 1002) sont déployées depuis le manchon de support externe (1022).

11. Dispositif de détection d'analytes (600, 1000) selon la revendication 10, dans lequel le manchon externe (1022) comprend en outre une barrière qui doit être percée par la pluralité d'électrodes de détection d'analytes (602, 1002) lorsqu'elles sont déplacées d'une position totalement rétractée à une position déployée.

12. Dispositif de détection d'analytes (600, 1000) selon l'une quelconque des revendications 1 à 11, comprenant en outre un processeur de signal pour moyenner la variation de signal sur la pluralité d'électrodes de détection d'analytes (602, 1002).
